# DEMANDE DE BREVET EUROPEEN

(11) **EP 1 946 741 A1**
(43) Date de publication de la demande: **23.07.2008**
(21) Numéro de dépôt: 07121861.4
(22) Date de dépôt: 29.11.2007
(51) Int. Cl.: A61K 8/04, A61K 8/31, A61K 8/36, A61K 8/44, A61Q 9/02

(54) **Mousse à raser sans savon à base de n-acylsarcosinate et d'acide gras saturé linéaire libre; procédé de rasage**

(30) Priorité: 17.01.2007 FR 0752725
(71) Demandeur: L'Oréal, 75008 Paris (FR)
(72) Inventeur: Aubert, Lionnel, 95270, ASNIERES SUR OISE (FR); Dussault, Lydia, 78860, ST NOM LE BRETECHE (FR)
(74) Mandataire: Miszputen, Laurent

(57) **Abrégé**

L'invention a pour objet une composition de rasage sans savon conditionnée dans un dispositif aérosol monobloc et produisant une mousse à la sortie du dispositif, caractérisée par le fait qu'elle comprend dans un milieu cosmétiquement acceptable
a) au moins une phase aqueuse
b) au moins une N-acylsarcosine où le radical acyle est en C₁₀-C₂₀ neutralisée de 50 à 90 % ;
c) au moins un acide gras linéaire saturé en C₁₄-C₁₈ non neutralisé ;
d) au moins un tensioactif non ionique
e) au moins un agent propulseur.

Elle se rapporte, en outre, à un procédé de rasage consistant à appliquer sur la surface de la peau à raser une composition telle que définie ci-dessus puis à raser les poils au moyen d'un rasoir.

L'invention concerne également un kit de rasage caractérisé par le fait qu'il comprend
a) au moins une composition telle que définie ci-dessus et
(b) au moins un rasoir notamment jetable et/ou
(c) un moyen d'étalement d'une composition de rasage.

## Description

La présente invention concerne donc une composition de rasage sans savon conditionnée dans un dispositif aérosol monobloc et produisant une mousse, comprenant au moins une N-acylsarcosine où le radical acyle est en C₁₀-C₂₀ neutralisée de 50 à 90% ; au moins un acide gras linéaire saturé en C₁₄-C₁₈ non neutralisé ; au moins un tensioactif non-ionique et au moins un agent propulseur.

La présente invention concerne également un procédé de rasage consistant à appliquer sur la peau une composition telle que définie ci-dessus.

Sur le marché des produits de rasage en aérosol coexistent les mousses à raser et les gels de rasage auto-moussants.

Les gels de rasage automoussants sont biens connus et ont été décrits, par exemple, dans les brevets US2,995,521 ; US3541,581, US4,405,489 ; US4,528,111; US4,651,503 ; US 5,248,495 ; US5,308,643 ; US5,326,556 et la demande de brevet WO91/07943. De telles formulations se présentent sous la forme d'une émulsion huile-dans-eau dans laquelle l'agent automoussant, généralement un hydrocarbure aliphatique volatil (ie : de faible point d'ébullition) est solubilisé dans la phase huileuse et la phase aqueuse comprend un savon hydrosoluble. Le produit est généralement conditionné dans un récipient aérosol avec une séparation telle qu'un piston ou une poche souple pour séparer l'agent automoussant de l'agent propulseur nécessaire pour l'expulsion du produit. Le produit est appliqué sous forme de gel transparent, translucide ou opaque qui est subtantiellement sans mousse jusqu'à l'étalement sur la peau, moment à partir duquel il se produit une mousse par évaporation de l'agent moussant hydrocarbure volatil.

Les mousses à raser sont en général sous la forme d'émulsion huile dans eau dans laquelle la phase aqueuse contient un savon hydrosoluble. Le produit est généralement conditionné dans un récipient aérosol monobloc dans lequel l'agent propulseur est en mélange avec le jus ; la mousse se forme à la sortie du dispositif aérosol. La conventionnelle mousse à raser avec savon peut assécher ou augmenter la rugosité de la peau (due à la présence de savon dans la formule). Pour atténuer cet effet les mousses à raser sont formulées en incorporant des agents adoucissants, par exemple des humectants, des émollients, des silicones. L'incorporation de ces produits influe sur la qualité esthétique du produit et peut provoquer aussi par usage répété un dessèchement de la peau.

Pour ces raisons se sont développés des produits de rasage « sans savon » qui dessèchent moins la peau avec des compositions contenant les N Acyl Sarcosinates. Ces matières premières utilisées sous leur forme neutralisée (par la soude ou la TEA) sont préconisées pour les produits de rasage aérosol. (Harry's Cosmeticology 7 th ed. 1982). Cependant ce type de formulation dites « sans savon » présentent des qualités de mousse à l'application très insuffisantes.

Il existe donc le besoin de trouver de nouvelles formulations de mousse à raser sans savon dont les qualités de mousse sont sensiblement améliorées sans les inconvénients énoncés précédemment.

Les N-acylsarcosinates sont des tensioactifs anioniques bien connus de formule: 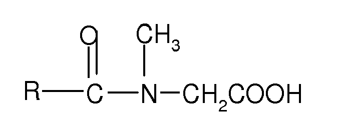 dans laquelle R est une chaîne hydrocarbonée d'acide gras. Ces tensioactifs sont généralement utilisés sous forme de sels hydrosolubles formés par neutralisation avec la soude, la potasse, l'ammoniaque ou la triéthanolamine et ont été préconisés dasn une large gamme de produits comme les shampooings, les détergents, les dentifrices, les crèmes à raser ou les savons pour les mains. Par exemple, des crèmes à raser aérosol contenant des sarcosinates sont décrites dans les brevets US3,959,160 ; US4,113,643 et US4,140,648 ainsi que dans le document Harry's Cosmetology (7 ème édition, 1982 page 169 -voir Croda Cosmetic and Pharmaceutical Formulary Supplement, formula SV11). Une crème non aérosol de rasage pouvant contenir un N-acylsarcosinate a été décrite dans le brevet US4,892,729 et un gel de rasage non aérosol contenant un savon et un sarcosinate a été décrit dans le brevet US5,340,571.

La demanderesse a découvert de manière surprenante que l'on pouvait obtenir des mousses à raser sans savon produisant à la sortie de l'aérosol une mousse de rigidité satisfaisante sans les inconvénients énoncés précédemment en utilisant une N-acylsarcosine neutralisée de 50 à 90% , au moins un acide gras linéaire saturé en C₁₄-C₁₈ non neutralisé et au moins un tensioactif non-ionique.

La présente invention concerne donc une composition de rasage sans savon conditionnée dans un dispositif aérosol monobloc et produisant une mousse à la sortie dudit dispositif, caractérisée par le fait qu'elle comprend dans un milieu cosmétiquement acceptable
a) au moins une phase aqueuse
b) au moins une N-acylsarcosine où le radical acyle est en C₁₀-C₂₀ neutralisée de 50 à 90%
c) au moins un acide gras linéaire saturé en C₁₄-C₁₈ non neutralisé;
d) au moins un tensioacif non-ionique
e) au moins un agent propulseur.

La présente invention concerne un procédé de rasage consistant à appliquer sur la peau une composition telle que définie ci-dessus.

L'invention concerne également un kit de rasage caractérisé par le fait qu'il comprend
a) au moins une composition telle que définie ci-dessus et
b) au moins un rasoir notamment jetable et/ou
c) un moyen d'étalement d'une composition de rasage.

On entend par « sans savon » comme contenant moins de 1% en poids de savon par rapport au poids total de la composition.

On entend par « acide gras non neutralisé », tout acide gras sous forme totalement libre.

On entend par « dispositif aérosol monobloc » comme dispositif pressurisé à un seul compartiment dans lequel le ou les agents propulseurs sont introduits avec le jus constituant la composition de rasage.

On entend par « milieu cosmétiquement acceptable » comme compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Les N-acylsarcosines conformes à l'invention sont choisies de préférence parmi celles ayant un radical acyle en C₁₂-C₁₈- Plus préférentiellement, elles sont choisies parmi stéaroyl sarcosine, myristoyl sarcosine, oleoyl sarcosine, lauroyl sarcosine, cocoyl sarcosine ou leurs mélanges. Encore plus préférentiellement, elles sont choisies parmi stéaroyl sarcosine, myristoyl sarcosine et leurs mélanges. La ou les sarcosines sont présents à des teneurs allant de 2% à 15% en poids et de préférence allant de 4 à 10% par rapport au poids total de la composition.

La N-acyl sarcosine conforme à l'invention est neutralisée de 50 à 90% par au moins une base minérale et/ou une base organique. La base peut être choisie parmi les bases minérales comme la potasse, la soude, l'ammoniaque. Elle peut être choisie parmi les bases organiques en particulier les alcanolamines telles que isopropanolamine, mono-, di- et triéthanolamine, aminoethylpropanol et aminomethylpropanol. La triéthanolamine est préférentielle. La quantité de base utilisée dépend de la quantité de sarcosine présente dans la composition.

Une quantité suffisante de base doit être utilisée pour neutraliser la sarcosine dans la phase aqueuse et produire un pH de 4 à 8.5 et plus préférentiellement de 5 à 7. Pour atteindre cette plage de pH, la sarcosine est de préférence neutralisée de 60 à 80%. On utilisera de préférence la sarcosine en léger excès molaire par rapport à la base. La base est de préférence présente à un taux variant de 1 à 6% par rapport au poids total de la composition.

Les acides gras saturés linéaires en C₁₄-C₁₈ sont choisis de préférence parmi l'acide myristique, palmitique, stéarique, cétylique ou leurs mélanges. Ils sont présents de préférence à des teneurs allant de 2% à 12% en poids et plus préférentiellement allant de 4 à 8% par rapport au poids total de la composition.

La phase aqueuse des compositions selon l'invention représente de préférence de 65 à 85% en poids du poids total de la composition et plus préférentiellement de 70 à 80% en poids.

Les tensioactifs non-ioniques utilisables selon l'invention sont bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools gras dont la chaîne grasse comporte de préférence de 8 à 2 atomes de carbone ; les alcools, les alpha-diols, les alkylphénols ou les acides gras polyéthoxylés, polypropoxylés ou polyglycérolés, ayant une chaîne grasse comportant, de préférence, de 8 à 20 atomes de carbone, et où le nombre de groupements oxyde d'éthylène ou oxyde de propylène varie de préférence de 2 à 60 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant de préférence en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de préférence de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-Cₗ₄)amines ou les oxydes de N-acyl(C₁₀-Cₗ₄)-aminopropylmorpholine ; et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, sous la dénomination PLANTAREN 2000 UP® par la société Cognis, et sous la dénomination ORAMIX NS 10® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; et le coco-glucoside comme le produit commercialisé sous la dénomination PLANTACARE 818/UP® par la société Cognis.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit fabriqué sous la dénomination CHIMEXANE NF® par la société Chimex.

Les tensioactifs non-ioniques préférentiels sont choisis parmi:
- les alcools gras ont la chaîne grasse est en C₈-C₂₀, plus préférentiellement dont la chaîne grasse est en C₁₂-C₁₈ comme par exemple l'alcool myristique, l'alcool laurique, l'alcool stéarylique et l'octyldodécanol ;
- les éthers polyoxyéthylénés d'alcools gras dont la chaîne grasse est en C₈-C₂₀, plus préférentiellement dont la chaîne grasse est en C₁₂-C₁₈ et ayant de 2 à 60, plus préférentiellement de 2 à 30 unités d'oxyde d'éthylène. Parmi ces composés, on peut citer par exemple Oleth-20, Steareth-21, Ceteth-20, Laureth-4, Laureth-23. De façon plus préférentielle, on choisira un mélange de Laureth-4 et Laureth-23.

Le ou les tensioactifs non-ioniques sont présents de préférence à des concentrations allant de 1 à 20% en poids et plus préférentiellement de 3 à 10% en poids par rapport au poids total de la composition.

L'agent propulseur est choisi de préférence parmi les hydrocarbures volatils et les hydrocarbures volatils halogénés. Le point d'ébullition de l'agent propuleur varie de préférence de -20 à 40°C. Les agents propulseurs utilisables selon l'invention sont choisis parmi les hydrocarbures aliphatiques en C₄-C₆ tels que n-pentane, isopentane, néopentane, n-butane, isobutane et leurs mélanges. Plus préférentiellement, on utilisera un mélange isopentane/butane/propane. L'agent propulseur est de préférence présent à des concentrations allant de 1 à 10% en poids et plus préférentiellement de 2 à 6% en poids par rapport au poids total de la composition.

Selon une forme préférentielle de l'invention, les compositions comportent en plus des tensioactifs choisis parmi les tensioactifs anioniques, les tensioactifs amphotères ou zwitterioniques et leurs mélanges.

On peut utiliser par exemple comme tensioactifs anioniques conformes à l'invention, les carboxylates, les alkyl sulfates oxyéthylénés ou non, les sulfonates, les alkyl sulfoacétates, les phosphates, les polypeptides, les dérivés anioniques d'alkyl polyglucoside, et leurs mélanges.

Comme carboxylates, on peut citer par exemple :
- les amido éthercarboxylates (AEC), comme le lauryl amido ether carboxylate de sodium (3 OE) commercialisé sous la dénomination AKYPO FOAM 30® par la société Kao Chemicals ;
- les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV® par la société Kao Chemicals ; les acides gras d'huile d'olive polyoxyéthylénés et de carboxyméthyle, produit commercialisé sous la dénomination OLIVEM 400® par la société Biologia E Tecnologia ; le tri-decyl éther carboxylate de sodium oxyéthyléné (6 OE) commercialisé sous la dénomination NIKKOL ECTD-6NEX® par la société Nikkol ;
- les acétates tels que le 2-(2-hydroxy alkyloxy)acétate de sodium commercialisé sous la dénomination BEAULIGHT SHAA par la société Sanyo ;
- les alaninates comme le N-lauroyl-N-methylamidopropionate de sodium, commercialisé sous la dénomination SODIUM NIKKOL ALANINATE LN 30® par la société Nikkol ou sous la dénomination ALANONE ALE® par la société Kawaken, et le N-lauroyl N-methylalanine triéthanolamine, commercialisé sous la dénomination ALANONE ALTA® par la société Kawaken ; (3) les acylglutamates comme le mono-cocoylglutamate de triéthanolamine commercialisé sous la dénomination ACYLGLUTAMATE CT-12® par la société Ajinomoto, et le lauroyl-glutamate de triéthanolamine commercialisé sous la dénomination ACYLGLUTAMATE LT-12® par la société Ajinomoto ; (4) les aspartates comme le mélange de N-lauroylaspartate de triéthanolamine et de N-myristoylaspartate de triéthanolamine, commercialisé sous la dénomination ASPARACK® par la société Mitsubishi ; (5) les glycinates comme le N-cocoyl glycinate de sodium commercialisé sous les dénominations AMILITE GCS-12® et AMILITE GCK 12 par la société Ajinomoto ;
- les citrates tels que le mono-ester citrique d'alcools de coco oxyéthylénés (9 OA) commercialisé sous la dénomination WITCONOL EC 1129 par la société Goldschmidt ;
- les galacturonates comme le Dodecyl D- galactoside uronate de sodium commercialisé par la société SOLIANCE ;

Comme alkyl sulfates oxyéthylénés ou non, on peut citer par exemple le lauryl éther sulfate de sodium (C12-14 70/30) (2,2 OE) commercialisé sous la dénomination SIPON AOS 225® par la société Cognis, le lauryl éther sulfate d'ammonium (C12-14 70/30) (3 OE) commercialisé sous la dénomination SIPON LEA 370® par la société Cognis, l'alkyl (C12-C14) éther (9 OE) sulfate d'ammonium commercialisé sous la dénomination RHODAPEX AB/20® par la société Rhodia Chimie, le mélange de lauryl et oleyl éther sulfate de sodium et magnésium commercialisé sous la dénomination EMPICOL BSD 52 par la société Albright & Wilson.

Comme sulfonates, on peut citer par exemple (1) les alpha-oléfines sulfonates comme l'alpha-oléfine sulfonate de sodium (C14-16) commercialisé sous la dénomination BIO-TERGE AS-40 pa s dénominations BIO-TERGE AS-40® et dénomination BIO-TERGE AS-40® par la société Stepan, sous les dénominations WITCONATE AOS PROTEGE® et SULFRAMINE AOS PH 12® par la société Witco, l'oléfine sulfonate de sodium secondaire commercialisé sous la dénomination HOSTAPUR SAS 30® par la société Clariant ; (2) les iséthionates comme le cocoyl-iséthionate de sodium, tel que le produit commercialisé sous la dénomination JORDAPON CI P® par la société Jordan, (3) les taurates comme le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo.

Comme phosphates, on peut citer par exemple les monoalkylphosphates et les dialkylphosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea.

Comme polypeptides (qui sont des composés obtenus par condensation d'une chaîne grasse sur les aminoacides de céréale et notamment du blé et de l'avoine), on peut citer par exemple le sel de potassium de la lauroyl protéine de blé hydrolysée, commercialisé sous la dénomination AMINOFOAM W OR® par la société Croda ; le sel de triéthanolamine de cocoyl protéine de soja hydrolysée, commercialisé sous la dénomination MAY-TEIN SY® par la société Maybrook ; le sel de sodium des lauroyl amino-acides d'avoine, commercialisé sous la dénomination PROTEOL OAT® par la société Seppic ; l'hydrolysat de collagène greffé sur l'acide gras de coprah, commercialisé sous la dénomination GELIDERM 3000® par la société Deutsche Gelatine ; les protéines de soja acylées par des acides de coprah hydrogénés, commercialisées sous la dénomination PROTEOL VS 22® par la société Seppic.

Comme dérivés anioniques d'alkyl-polyglucosides, on peut citer notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia ; le sel di-sodique d'ester sulfosuccinique de cocoylpolyglucoside (1,4), commercialisé sous la dénomination ESSAI 512 MP® par la société Seppic ; le sel de sodium d'ester citrique de cocoyl polyglucoside (1,4) commercialisé sous la dénomination EUCAROL AGE-EC® par la société Cesalpinia, le Lauryl polyglucoside éther carboxylate de sodium commercialisé sous la dénomination PLANTAPON LGC SORB par la société Cognis.

On utilisera de préférence les sels d'alkyléthersulfates en C₆-C₂₄ comportant de 1 à 30 groupements oxyde d'éthylène , en particulier les sels de métaux alcalins ou alcalino-terreux, d'ammonium, d'amine ou d'aminoalcool et plus particulièrement les sels de sodium et encore plus particulièrement les alkyl (C₁₂-C₁₄)éthersulfates de sodium oxyéthylénés comportant un nombre moyen de groupes oxyde d'éthylène compris entre 1 et 4 et plus particulièrement le Sodium Laureth Sulfate (nom CTFA).

On peut utiliser par exemple comme tensioactifs amphotères ou zwitterioniques, les alkylbétaïnes, les N-alkylamidobétaïnes et leurs dérivés, les sultaïnes, les alkyl-polyaminocarboxylate (APAC), les alkylamphoacétates et leurs mélanges.

Comme alkylbétaïnes, on peut citer par exemple la cocobétaïne comme le produit commercialisé sous la dénomination DEHYTON AB-30® par la société Cognis ; la laurylbétaïne comme le produit commercialisé sous la dénomination GENAGEN KB® par la société Clariant ; la laurylbétaïne oxyethylénée (10 OE), comme le produit commercialisé sous la dénomination LAURYLETHER (10 OE) BETAINE® par la société Shin Nihon Rica ; la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou sous la dénomination EMPIGEN BB® par la société Albright & Wilson ; la lauramidopropyl bétaïne commercialisée sous la dénomination REWOTERIC AMB12P® par la société Witco.

Comme sultaines, on peut citer le cocoyl-amidopropylhydroxy-sulfobetaine commercialisé sous la dénomination CROSULTAINE C-50® par la société Croda.

Comme alkyl-polyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel ; le stéaryl-polyamidocarboxylate de sodium commercialisé sous la dénomination AMPHOLAK 7 TX/C® par la société Akzo Nobel ; la carboxyméthyloléyl-polypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP® par la société Rhodia Chimie ; et le N-cocoyl-N-hydroxyéthyl-N-carboxyméthyl-éthylènediamine N-sodique (nom CTFA : sodium cocamphoacetate).

On utilisera plus particulièrement parmi les tensio-actifs amphotères
- la cocobétaine tels que les produits commerciaux MIRATAINE BB/FLA de RHODIA ou EMPIGEN BB/FL de Huntsman ;
- la Lauroybetaine (and) Sodium Chloride tels que les produits commerciaux EMPIGEN BB/LS de Huntsman
- le cocoamphodiacétate de disodium comme le produit commercialisé sous la dénomination commerciale MIRANOL® C2M concentré par la société RHODIA.
ou leurs mélanges.

Le ou les tensioactifs anioniques et/ou amphotères ou zwitterioniques sont présents de préférence à des concentrations allant de 1 à 10% en poids et plus préférentiellement de 2 à 7% en poids par rapport au poids total de la composition.

Les compositions de l'invention peuvent également contenir un agent gélifiant hydrosoluble ou un agent épaississant pour améliorer la consistance et la stabilité de la mousse.

Parmi ces agents gelifiants, on peut citer les polymères hydroxyalkylcelluloses comme hydroxyethylcellulose ou hydroxypropylcellulose (produits vendus respectivement sous la dénomination commerciale NATROSOL ou KLUCEL) ; les copolymères d'acide acrylique et de polyallylsucrose (produits vendus sous la dénomination commerciale CARBOPOL) ; la carboxymethylcellulose et la cellulose methyl ether (produits vendus sous la dénomination commerciale METHOCEL) ; les gommes naturelles ou synthétiques, les amidons. Les agents épaississants préférentiels sont choisis parmi hydroxyethylcellulose ou hydroxypropylcellulose ou leurs mélanges. Les agents gelifiants auxiliaires ou épaississants sont de préférence présents à des concentrations allant de 0,01 à 5% en poids, plus préférentiellement de 0,05 à 2% en poids par rapport au poids total de la composition.

Les compositions de l'invention peuvent contenir en plus un polyol à chaîne courte pour améliorer les qualités de mousse et/ou la stabilité de la composition. Le ou les polyols sont de préférence présents à des concentrations inférieures ou égale à 15% en poids et plus préférentiellement allant de 0,25 à 10% en poids par rapport au poids total de la composition. Parmi les polyols utilisables ont peut citer la glycérine, le propylène glycol ou leurs mélanges.

Les compositions de rasage selon l'invention peuvent contenir en plus une variété d'ingrédients cosmétiques classiques pour améliorer les qualités esthétiques et les performances de ces compositions.

Les compositions selon l'invention peuvent également contenir en plus un polymère cationique conditionneur pour améliorer la lubricité et le toucher de la peau après rasage. On peut citer par exemple les sels d'ammonium quaternaires de l'hydroxyyethylcellulose comme Polyquaternium-10, Polyquaternium-24.

On peut également citer les polymères cationiques suivants :
Polyquaternium 5 tel que le produit MERQUAT 5 commercialisé par la société CALGON ;
Polyquaternium 6 tel que le produit SALCARE SC 30 commercialisé par la société CIBA, et le produit MERQUAT 100 commercialisé par la société CALGON ;
Polyquaternium 7 tel que les produits MERQUAT S, MERQUAT 2200 et MERQUAT 550 commercialisés par la société CALGON, et le produit SALCARE SC 10 commercialisé par la société CIBA ;
Polyquaternium 11 tel que les produits GAFQUAT 755, GAFQUAT 755N et GAFQUAT 734 commercialisés par la société ISP ;
Polyquaternium 15 tel que le produit ROHAGIT KF 720 F commercialisé par la société ROHM ;
Polyquaternium 16 tel que les produits LUVIQUAT FC905, LUVIQUAT FC370, LUVIQUAT HM552 et LUVIQUAT FC550 commercialisés par la société BASF ;
Polyquaternium 22 tel que le produit MERQUAT 280 commercialisé par la société CALGON ;
Polyquaternium 28 tel que le produit STYLEZE CC10 commercialisé par la société ISP;
Polyquaternium 39 tel que le produit MERQUAT PLUS 3330 commercialisé par la société CALGON ;
Polyquaternium 44 tel que le produit LUVIQUAT CARE commercialisé par la société BASF;
Polyquaternium 46 tel que le produit LUVIQUAT HOLD commercialisé par la société BASF;
Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

Le ou les polymères cationiques conditionneurs sont présents de préférence à des concentrations allant de 0,05 à 2% en poids, plus préférentiellement allant de 0,1 à 1% en poids par rapport au poids total de la composition.

D'autres additifs peuvent également utilisés dans les compositions de l'invention comme
- les humectants comme le sorbitol ;
- les émollients tels que les esters gras comme l'isopropyl myristate, le decyl oleate, le 2-ethyhexyl palmitate, le PEG-7 Glyceryl Cocoate et le Glyceryl Linoleate ; les éthers gras propoxylés comme PPG-10 Cetyl Ether et PPG-11 Stearyl Ether ; les di- ou triglycérides comme la lécithine, le mélange de triglycérides caprique/caprylique, le PEG-10 Soy Sterol, les huiles végétales.
- les agents rafraichissants et les agents apaisants comme le menthol, l'aloe, l'allantoine, la lanoline, le bisabolol, l'acide hyaluronique ;
- les lubrifiants comme les polyethyleneglycols (ie PEG-14M, PEG-23M), les tensioactifs fluorés, les silicones (ie : dimethicone, dimethiconol, dimethicone copolyol, stearyl dimethicone, cetyl dimethicone copolyol, cyclomethicone, etc...)
- les vitamines incluant les précurseurs et les dérivés comme le panthenol, le tocopheryl acetate, le niacinamide, le retinyl palmitate, le vitamine A palmitate ;
- les charges
- les colorants;
- les parfums ;
- les antioxydants;
- les antibactériens et/ou les antifongiques;
- les conservateurs (ie : methylchloroisothiazolinone, methylisothiazolinone, DMDM hydantoine, iodopropinyl butylcarbamate).

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyl-lysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions de rasage de la présente invention sont conditionnées dans un dispositif aérosol monobloc (à un seul compartiment) dans lequel l'agent propulseur et le jus sont mélangés.

L'invention concerne également un kit de rasage caractérisé par le fait qu'il comprend
a) au moins une mousse à raser telle que définie ci-dessus et
b) au moins un rasoir notamment jetable et/ou
c) un moyen d'étalement d'une composition de rasage.

Selon une forme particulière de l'invention, le kit peut contenir en plus une composition après-rasage pour apaiser le feu du rasage.

Les exemples qui suivent servent à illustrer l'invention. Les quantités indiquées sont en % en poids sauf mention contraire, et les noms des composés en noms chimiques ou noms CTFA (International Cosmetic Ingredient Dictionary and Handbook) selon les cas.

### EXEMPLE

On prépare les mousses à raser sans savon suivantes 1 à 9 conditionnées dans des aérosols monoblocs. Les quantités sont indiquées en % en poids par rapport au poids total de la composition.

| **Ingrédients** | **Ex 1 (*)** | **Ex 2 (*)** | **Ex 3 (*)** | **Ex 4** | **Ex 5** |
|---|---|---|---|---|---|
| 2,6-dimethyl-7-octen-2-ol | 0,29 | 0,29 | 0,29 | 0,29 | 0,29 |
| Huile de paraffine | - | 2,87 | - | - | - |
| Acide laurique (C₁₂) | - | - | 2,87 | - | - |
| Acide myristique (C₁₄) | - | - | - | 2,87 | - |
| Acide palmitique (C₁₆) | - | - | - | - | 2,87 |
| Glycérine | 4,80 | 4,80 | 4,80 | 4,80 | 4,80 |
| Polyquaternium-7 (MERQUAT S) | 0,48 | 0,48 | 0,48 | 0,48 | 0,48 |
| Hydroxyethylcellulose (PM : 1.000.000) | 1,00 | 1,00 | 1,00 | 1,00 | 1,00 |
| Isobutane/propane/butane (56/24/20) | 4,25 | 4,25 | 4,25 | 4,25 | 4,25 |
| Stearoyl sarcosine (and) Myristoyl sarcosine (75%/25%) | 2,87 | 2,87 | 2,87 | 2,87 | 2,87 |
| Triéthanolamine | 1,19 | 1,19 | 1,19 | 1,19 | 1,19 |
| Dimethiconol Stearate (MIRASIL WAX-S de RHODIA) | 0,67 | 0,67 | 0,67 | 0,67 | 0,67 |
| Steareth-100 | 0.48 | 0.48 | 0.48 | 0.48 | 0.48 |
| Laureth-23 | 4.80 | 4.80 | 4.80 | 4.80 | 4.80 |
| Gomme de Xanthane (RHODICARE CFT de RHODIA) | 1,00 | 1,00 | 1,00 | 1,00 | 1,00 |
| Lauryl Betaine (and) Sodium Chloride (EMPIGEN BB/LS de Huntsman) | 4,80 | 4,80 | 4,80 | 4,80 | 4,80 |
| Conservateurs | qs | qs | qs | qs | qs |
| Eau | qsp 100 g | qsp 100 g | qsp 100 g | qsp 100 g | qsp 100 g |
| **Rigidité de la mousse à raser (en grammes)** | **3** | **3** | **4,2** | **11 ,5** | **11,5** |

| **Ingrédients** | | **Ex 6** | **Ex 7 (*)** | **Ex 8 (*)** | **Ex 9 (*)** |
|---|---|---|---|---|---|
| 2,6-dimethyl-7-octen-2-ol | | 0,29 | 0,29 | 0,29 | 0,29 |
| Acide stéarique (C₁₈) | | 2,87 | | | |
| Acide isostéarique (C₁₈ ramifié) | | - | 2,87 | - | - |
| Acide oléique (C₁₈ insaturé) | | - | - | 2,87 | - |
| Acide béhénique (C₂₀/C₂₁) | | | | | 2,87 |
| Glycérine | | 4,80 | 4,80 | 4,80 | 4,80 |
| Polyquaternium-7 (MERQUAT S) | | 0,48 | 0,48 | 0,48 | 0,48 |
| Hydroxyethylcellulose (PM : 1.000.000) | | 1,00 | 1,00 | 1,00 | 1,00 |
| Isobutane/propane/butane (56/24/20) | | 4,25 | 4,25 | 4,25 | 4,25 |
| Stearoyl sarcosine (and) Myristoyl sarcosine (75%/25%) | | 2,87 | 2,87 | 2,87 | 2,87 |
| Triéthanolamine | | 1,19 | 1,19 | 1,19 | 1,19 |
| Dimethiconol Stearate (MIRASIL WAW-S de RHODIA) | | 0,67 | 0,67 | 0,67 | 0,67 |
| Steareth-100 | | 0.48 | 0.48 | 0.48 | 0.48 |
| Laureth-23 | | 4.80 | 4.80 | 4.80 | 4.80 |
| Gomme de Xanthane (RHODICARE CFT de RHODIA) | | 1,00 | 1,00 | 1,00 | 1,00 |
| Lauryl Betaine (and) Sodium Chloride (EMPIGEN BB/LS de Huntsman) | | 4,80 | 4,80 | 4,80 | 4,80 |
| Conservateurs | | qs | qs | qs | qs |
| Eau | | qsp 100 g | qsp 100 g | qsp 100 g | qsp 100 g |
| **Rigidité de la mousse à raser (en grammes)** | | **12** | **3,6** | **3** | **3,1** |

| | | | | | |
|---|---|---|---|---|---|
| (*) hors invention (*) hors invention | | | | | |

On mesure à 25°C la rigidité de chaque mousse à raser au moyen d'un analyseur de texture TA XT2i fabriqué par la société THERMO muni d'un cylindre SMS P/0-5 HS 0.5 inch diameter Hemi Spherical Delrin Cylinder Probe. On mesure la rigidité (exprimée en grammes) de chaque produit en compression par ledit cylindre à une vitesse de 2 mm/s sur une distance de 25 mm.

On observe que l'huile de paraffine, l'acide laurique (C12), l'acide oléique (insaturée en C₁₈), l'acide isostéarique (acide gras ramifié en C₁₈) et l'acide béhénique (acide gras linéaire en C₂₀/C₂₁) des exemples 2, 7, 8 et 9 conduisent à des mousses à raser de faible rigidité (inférieure ou égale à 3,6). Ces huiles n'apportent pas ou pratiquement pas d'amélioration par rapport à l'exemple 1 ne contenant pas d'acide gras libre.

On observe que les acides gras linéaires saturés en C₁₄-C₁₈ des exemples 4, 5 et 6 de l'invention conduisent de manière surprenante à des mousses de plus forte rigidité (ie 11,5 ou 12 g).

## Revendications

1. Composition de rasage sans savon conditionnée dans un dispositif aérosol monobloc et produisant une mousse à la sortie dudit dispositif, **caractérisée par le fait qu'**elle comprend dans un milieu cosmétiquement acceptable
a) au moins une phase aqueuse
b) au moins une N-acylsarcosine où le radical acyle est en C₁₀-C₂₀ neutralisée de 50 à 90 % ;
c) au moins un acide gras linéaire saturé en C₁₄-C₁₈ non neutralisé;
d) au moins un tensioacif non-ionique
e) au moins un agent propulseur.

2. Composition selon la revendication 1, où la N-acylsarcosine a un radical acyle en C₁₂-C₁₈.

3. Composition selon la revendication 1 ou 2, où la N-acylsarcosine est choisie parmi stéaroyl sarcosine, myristoyl sarcosine, oleoyl sarcosine, lauroyl sarcosine, cocoyl sarcosine ou leurs mélanges.

4. Composition selon la revendication 3, où la N-acylsarcosine est choisie parmi stéaroyl sarcosine, myristoyl sarcosine et leurs mélanges.

5. Composition selon l'une quelconque des revendications 1 à 4, où la ou les N-acylsarcosines sont présentes à des teneurs allant de 2% à 15% en poids et de préférence allant de 4 à 10% par rapport au poids total de la composition.

6. Composition selon l'une quelconque des revendications 1 à 5, où la sarcosine est neutralisée de 60 à 80%.

7. Composition selon l'une quelconque des revendications 1 à 6, où la phase aqueuse des compositions selon l'invention représente de préférence de 65 à 85% en poids du poids total de la composition et plus préférentiellement de 70 à 80% en poids.

8. Composition selon l'une quelconque des revendications 1 à 7, où le ou les acides gras saturés linéaires en C₁₄-C₁₈ sont choisis parmi l'acide myristique, palmitique, stéarique, cétylique ou leurs mélanges.

9. Composition selon l'une quelconque des revendications 1 à 8, où le ou les acides gras saturés linéaires en C₁₄-C₁₈ sont présents à des teneurs allant de 2% à 12% en poids et de préférence allant de 4 à 8% par rapport au poids total de la composition.

10. Composition selon l'une quelconque des revendications 1 à 9, où le tensioactif non-ionique est choisi parmi les alcools gras ; les alcools, les alpha-diols, les alkylphénols ou les acides gras polyéthoxylés, polypropoxylés ou polyglycérolés ; les copolymères d'oxyde d'éthylène et de propylène ; les condensats d'oxyde d'éthylène et de propylène sur des alcools gras ; les amides gras polyéthoxylés ; les amides gras polyglycérolés ; les amines grasses polyéthoxylées ; les esters d'acides gras du sorbitane éthoxylés; les esters d'acides gras du saccharose, les esters d'acides gras du polyéthylèneglycol ; les alkyl(C₆-C₂₄)polyglycosides ; les dérivés de N-alkyl(C₆-C₂₄)glucamine ; les oxydes d'amines et leurs mélanges.

11. Composition selon la revendication 10, où le ou les tensioactifs non-ioniques sont choisis parmi :
- les alcools gras dont la chaîne grasse est en C₈-C₂₀, plus préférentiellement dont la chaîne grasse est en C₁₂-C₁₈ ;
- les éthers polyoxyéthylénés d'alcools gras dont la chaîne grasse est en C₈-C₂₀, plus préférentiellement dont la chaîne grasse est en C₁₂-C₁₈ et ayant de 2 à 60, plus préférentiellement de 2 à 30 unités d'oxyde d'éthylène
et leurs mélanges

12. Composition selon l'une quelconque des revendications 1 à 11, où le ou les tensioactifs non-ioniques sont présents à des concentrations allant de 1 à 20% en poids et plus préférentiellement de 3 à 10% en poids par rapport au poids total de la composition.

13. Composition selon l'une quelconque des revendications 1 à 12, où l'agent propulseur est choisi parmi les hydrocarbures volatils et les hydrocarbures volatils halogénés.

14. Composition selon la revendication 13, où l'agent propulseur est choisi parmi les hydrocarbures aliphatiques en C₄-C₆.

15. Composition selon la revendication 14, où l'agent propulseur est choisi parmi le n-pentane, isopentane, néopentane, n-butane, isobutane et leurs mélanges.

16. Composition selon la revendication 15, où l'agent propulseur est un mélange isopentane/butane/propane.

17. Composition selon l'une quelconque des revendications 1 à 16, où l'agent propulseur est présent à des concentrations allant de 1 à 10% en poids et plus préférentiellement de 2 à 6% en poids par rapport au poids total de la composition.

18. Composition selon l'une quelconque des revendications 1 à 17, comprenant en plus des tensioactifs choisis parmi les tensioactifs anioniques, les tensioactifs amphotères ou zwitterioniques et leurs mélanges.

19. Composition selon la revendication 18, où le tensioactif anionique est choisi parmi les carboxylates, les alkyl sulfates oxyéthylénés ou non, les sulfonates, les alkyl sulfoacétates, les phosphates, les polypeptides, les dérivés anioniques d'alkyl polyglucoside, et leurs mélanges.

20. Composition selon la revendication 18, où le tensioactif anionique est choisi parmi les sels d'alkyléthersulfates en C₆-C₂₄ comportant de 1 à 30 groupements oxyde d'éthylène et plus particulièrement les sels d'alkyl (C₁₂-C₁₄)éthersulfates de sodium oxyéthylénés comportant un nombre moyen de groupes oxyde d'éthylène compris entre 1 et 4.

21. Composition selon la revendication 18, où le tensioactif amphotère ou zwitterionique est choisi parmi les alkylbétaïnes, les N-alkylamidobétaïnes et leurs dérivés, les sultaïnes, les alkyl-polyaminocarboxylate (APAC), les alkylamphoacétates et leurs mélanges.

22. Composition selon l'une quelconque des revendications 1 à 21, où le ou les tensioactifs anioniques et/ou amphotères ou zwitterioniques sont présents à des concentrations allant de 1 à 10% en poids et plus préférentiellement de 2 à 7% en poids par rapport au poids total de la composition.

23. Composition selon l'une quelconque des revendications 1 à 22, contenant en plus au moins un agent gélifiant hydrosoluble et/ou au moins un agent épaississant.

24. Composition selon la revendication 23, où l'agent gélifiant auxiliaire hydrosoluble ou l'agent épaississant sont choisis parmi les polymères hydroxyalkylcelluloses ; les copolymères d'acide acrylique et de polyallylsucrose ; la carboxymethylcellulose et la cellulose méthyl ether ; les gommes naturelles ou synthétiques, les amidons.

25. Composition selon la revendication 23 ou 24, où l'agent gélifiant auxiliaire hydrosoluble et/ou l'agent épaississant sont présents à des concentrations allant de 0,05 à 5% en poids, plus préférentiellement de 0,01 à 2% par rapport au poids total de la composition.

26. Composition selon l'une quelconque des revendications 1 à 25, contenant en plus au moins un polyol à chaîne courte

27. Composition selon la revendication 26, où le ou les polyols sont présents à des concentrations inférieures à 5% en poids et plus préférentiellement allant de 0,25 à 5% en poids par rapport au poids total de la composition.

28. Composition selon la revendication 26 ou 27, où le ou les polyols sont choisis parmi la glycérine, le propylène glycol ou leurs mélanges.

29. Composition selon l'une quelconque des revendications 1 à 28, contenant en plus au moins un additif choisi parmi
- les polymères cationiques conditionneurs,
- les humectants,
- les émollients,
- les charges,
- les agents rafraîchissants et les agents apaisants
- les lubrifiants
- les vitamines incluant les précurseurs et les dérivés
- les colorants ;
- les parfums ;
- les antioxydants;
- les antibactériens et/ou les antifongiques;
- les conservateurs.

30. Procédé de rasage consistant à appliquer sur la surface de la peau à raser une composition telle que définie dans l'une quelconque des revendications précédentes puis à raser les poils au moyen d'un rasoir.

31. Kit de rasage **caractérisé par le fait qu'**il comprend
a) au moins une composition telle que définie dans l'une quelconque des revendications précédentes (b) au moins un rasoir notamment jetable et/ou
(c) un moyen d'étalement d'une composition de rasage.

32. Kit de rasage selon la revendication 31, contenant en plus une composition après-rasage pour apaiser le feu du rasage.
